(19) Europäisches Patentamt
European Patent Office
Office européen des brevets

(11) **EP 2 218 438 B1**

(12) **FASCICULE DE BREVET EUROPEEN**

(45) Date de publication et mention
de la délivrance du brevet:
**20.12.2017 Bulletin 2017/51**

(51) Int Cl.:
*A61K 8/34* *(2006.01)*    *A61K 8/39* *(2006.01)*
*A61K 8/86* *(2006.01)*    *A61Q 15/00* *(2006.01)*
*A61K 8/36* *(2006.01)*    *A61K 8/02* *(2006.01)*

(21) Numéro de dépôt: **10150888.5**

(22) Date de dépôt: **15.01.2010**

(54) **Composition solide transparente parfumante à base de sels d'acide gras et d'un alcool gras oxyethylene ; procédé de stabilisation**

Solid Deodorant Zusammensetzung umfassend Fettsäuresalze und ethoxylierten Fettalkohol; Stabilisierung Methode

Solid deodorant composition comprising fatty acid salts and ethoxylated fatty alcohol; stabilisation method

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **17.02.2009 FR 0951007**

(43) Date de publication de la demande:
**18.08.2010 Bulletin 2010/33**

(73) Titulaire: **L'Oréal**
**75008 Paris (FR)**

(72) Inventeurs:
 • **Cassier, Matthieu**
  **75017, Paris (FR)**

 • **Lefebvre, Fanny**
  **94340, Joinville Le Pont (FR)**

(74) Mandataire: **Kromer, Christophe**
**L'Oréal**
**Service DIPI**
**9 Rue Pierre Dreyfus**
**92110 Clichy (FR)**

(56) Documents cités:
**US-A- 5 407 668    US-A- 5 424 070**
**US-A- 5 863 524    US-A1- 2003 206 931**

**Description**

L'invention concerne une composition solide transparente aqueuse parfumante dans un support cosmétiquement acceptable :

a) au moins une phase aqueuse et

b) au moins un sel d'acide gras (savon) dans une quantité de 0,5 à 20% en poids par rapport au poids total de la composition et

c) au moins un alcool gras oxyéthyléné de formule (I') que l'on définira plus loin en détail dans une quantité allant de 0,1 à 10% en poids par rapport au poids total de la composition et

d) et au moins une substance parfumante dans une quantité allant de 0,5 à 30% en poids par rapport au poids total de la composition.

Dans le domaine cosmétique, il est bien connu d'utiliser en application topique, des produits solides transparents ou translucides (sticks) notamment dans le domaine de l'hygiène corporelle comme les sticks déodorants.

Les sticks transparents ou translucides aqueux à base de sels d'acide gras ou savons sont particulièrement recherchés pour leurs qualités cosmétiques et leur aspect transparent qui évoque pour le consommateur à la fois la clarté, la propreté et l'efficacité du produit. Ils ont été notamment décrits dans les brevets US4,559,924 ; US5,114,717 ; US5,120,541, US4,440,742 ; US5,128,123 ; US4,504,465 ; US4,226,889, US4,702,916 ; US4,732,754. Il est important que l'aspect du stick (ie : homogénéité, transparence) puisse rester stable dans le temps dans les conditions de stockage et d'utilisation.

On connaît dans le brevet US5,863,524 des sticks déodorants de transparence stable dans le temps à base de sels d'acide gras, de sel de bicarbonate et de métal alcalin et un agent clarifiant du type polyamine. On connaît également des sticks aqueux de transparence stable dans le temps à base de sels d'acide gras, d'un alcool gras polyoxyalkylé du type Eumulgin comme le Ceteareth-30 ou le PPG-2-ceteareth-9. On a également proposé dans le brevet US5,407,668 d'utiliser un mélange constitué d'un alcanolamide et d'un alcool gras monoalcoxylé pour renforcer la transparence et la stabilité de la transparence dans des sticks déodorants à base de savons.

Pour le confort et l'agrément l'utilisateur, les sticks aqueux transparents à base de sels d'acide gras comprennent généralement en plus des parfums. Au cours de ses recherches, la demanderesse a constaté que la présence du parfum pouvait conduire à une forte augmentation de l'indice d'acide et à la formation dans le temps de cristaux au sein de la matrice du stick et rendre celui-ci hétérogène, trouble, opaque.

Aussi, il subsiste donc le besoin de rechercher de nouvelles compositions solides parfumées aqueuses à base de sels d'acide gras ne présentant pas les inconvénients des produits de l'art antérieur à savoir dont l'homogénéité et/ou la transparence restent stables dans le temps.

La demanderesse a découvert d'une manière surprenante que l'on pouvait atteindre cet objectif en utilisant un alcool gras oxyéthyléné de formule (I) que l'on définira plus loin en détail dans une composition solide à base de sels d'acide gras et comprenant au moins une substance parfumante.

Cette découverte constitue la base de l'invention.

La présente invention concerne donc une composition solide transparente aqueuse parfumante comprenant dans un support cosmétiquement acceptable :

a) au moins une phase aqueuse et

b) au moins un sel d'acide gras dans une quantité allant de 0,5 à 20% en poids par rapport au poids total de la composition et

c) au moins un alcool gras oxyéthyléné de formule (I') que l'on définira plus loin en détail dans une quantité allant de 0,1 à 10% en poids par rapport au poids total de la composition et

d) au moins une substance parfumante dans une quantité allant de 0,5 à 30% en poids par rapport au poids total de la composition.

La présente invention concerne également un procédé de stabilisation d'une composition solide transparente aqueuse parfumante comprenant dans un support cosmétiquement acceptable :

a) au moins une phase aqueuse et

b) au moins un sel d'acide gras dans une quantité allant de 0,5 à 20% en poids par rapport au poids total de la composition et

c) au moins une substance parfumante dans une quantité allant de 0,5 à 30% en poids par rapport au poids total de la composition, caractérisé par le fait qu'il consiste à ajouter dans ladite composition au moins un alcool gras polyoxyéthyléné de formule (I') que l'on définira plus loin en détail, dans une quantité allant de 0,1 à 10% en poids par rapport au poids total de la composition.

D'autres objets de l'invention apparaîtront à la suite de la description.

Par composition parfumante, on entend toute composition laissant après application sur les matières kétatiniques un parfum.

Par « substance parfumante », on entend tout parfum ou arôme susceptible de parfumer la peau et les matières kératiniques humaines en général comprenant la peau, les cheveux, le cuir chevelu, les lèvres ; les ongles.

On entend par « support cosmétiquement acceptable » un milieu non toxique et susceptible d'être appliqué sur les matières kératiniques humaines comprenant la peau, le visage, les lèvres, les ongles, les cheveux, le cuir chevelu.

On entend par "transparente " une composition présentant une turbidité inférieure à 400 UTN (Unités de Turbidité Nephélométique) à 25°C et de préférence inférieure à 250 UTN à 25°C, mesurée avec un appareil 2100P Turbidimeter de la société HACH.

Par « composition solide », on entend que la mesure de la force maximale mesurée en texturométrie lors de l'enfoncement d'une sonde dans l'échantillon de formule doit être au moins égale à 0,25 Newton, en particulier au moins égal à 0,30 Newton, notamment au moins égale 0,35 Newton, appréciée dans des conditions de mesure précises comme suit.

Les formules sont coulées à chaud dans des pots de 4 cm de diamètre et 3 cm de fond. Le refroidissement est fait à température ambiante. La dureté des formules réalisées est mesurée après 24 heures d'attente. Les pots contenant les échantillons sont caractérisés en texturométrie à l'aide d'un texturomètre tel que celui commercialisé par la société Rhéo TA-XT2, selon le protocole suivant : une sonde de type bille en inox de diamètre 5 mm est amenée au contact de l'échantillon à une vitesse de 1 mm/s. Le système de mesure détecte l'interface avec l'échantillon avec un seuil de détection égal à 0,005 newtons. La sonde s'enfonce de 0,3 mm dans l'échantillon, à une vitesse de 0,1 mm/s. L'appareil de mesure enregistre l'évolution de la force mesurée en compression au cours du temps, pendant la phase de pénétration. La dureté de l'échantillon correspond à la moyenne des valeurs maximales de la force détectée pendant la pénétration, sur au moins 3 mesures.

## SAVONS

Le sel d'acide gras ou savon est obtenu à partir d'un acide gras et d'une base, l'acide gras comportant une chaîne alkyle linéaire ou ramifiée, saturée ou insaturée, ayant de 12 à 22 atomes de carbone et de préférence 12 à 20 atomes de carbone.

Les bases (aussi appelées aussi agents de saponification) neutralisent totalement ou partiellement les acides gras. Les bases susceptibles d'être utilisées pour obtenir les sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium, ou encore les bases organiques comme la triéthanolamine, la N-méthylglucamine, la lysine et l'arginine. Selon un mode particulier de réalisation de l'invention, la base est la potasse.

Le savon est généralement introduit dans la composition sous forme de la base d'une part et de l'acide gras d'autre part, la formation du sel se faisant in situ.

Le taux de neutralisation de l'acide gras est défini comme étant le rapport massique entre les acides gras sous forme de sels et les acides gras totaux (acides libres + sels d'acides gras). Dans la composition selon l'invention, ce taux va de préférence de 80 à 97 %, et mieux de 85 à 95 %.

L'acide gras peut être choisi en particulier parmi les acides gras en $C_{10}$ à $C_{24}$, et notamment en $C_{12}$-$C_{18}$, et en particulier l'acide laurique, l'acide myristique, l'acide stéarique, l'acide palmitique et leurs mélanges.

Comme savons, on peut citer par exemple les sels de potassium et les sels de sodium des acides gras en $C_{10}$-$C_{24}$, notamment en $C_{12}$-$C_{20}$, plus spécialement en $C_{12}$-$C_{18}$. Le savon peut être plus spécialement choisi parmi les sels de sodium des acides gras en $C_{12}$-$C_{18}$, plus spécialement le sel de sodium de l'acide stéarique.

La quantité totale de sels d'acide gras dans la composition de l'invention varie de 1 à 10 % en poids par rapport au poids total de la composition.

## ALCOOL GRAS OXYETYLENE

Les alcools béhéniques oxyéthylénés conformes à l'invention répondent à la formule générale (I') suivante :

$$CH_3(CH_2)_{20}\text{-}CH_2\text{-}(OCH_2CH_2)_y\text{-}OH \qquad (I')$$

dans laquelle y varie de 1 à 20 et plus préférentiellement de 1 à 10.

Parmi les alcools béhéniques oxyéthylénés, on peut citer les composés suivants Beheneth-1, Beheneth-2, Beheneth-3, Beheneth-4, Beheneth-5, Beheneth-6, Beheneth-7, Beheneth-8, Beheneth-9, Beheneth-10, Beheneth-11, Beheneth-12, Beheneth-13, Beheneth-14, Beheneth-15, Beheneth-16, Beheneth-17, Beheneth-18, Beheneth-19, Beheneth-20.

On peut citer comme produits commerciaux :

- les produits commercialisés sous les noms EMULGIN BA (+ le nombre d'ethoxylation) par la société COGNIS comme par exemple EUMULGIN BA 10 pour le Beheneth-10, EUMULGIN BA 20 pour le Beheneth-20 ;

- les produits commercialisés sous les noms NIKKOL BB (+le nombre d'ethoxylation) par la société NIKKO CHEMICALS comme par exemple NIKKOL BB 20 pour le Beheneth-20 ;

- les produits commercialisés sous les noms EMALEX-BHA par la société NIHON EMULSION comme par exemple EUMULGIN BA 10 pour le Beheneth-10.

On utilisera plus particulièrement le Beheneth-10.

La quantité d'alcool gras oxyéthyléné de formule (I) varie de 0,1 à 10% en poids et plus préférentiellement de 0,5 à2% en poids par rapport au poids total de la composition.

## PARFUMS

Comme substance parfumante, on peut utiliser dans la composition de l'invention, les parfums et les arômes d'origine naturelle ou synthétique et leurs mélanges. Comme parfums et arômes d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme substance parfumante d'origine synthétique, on peut citer par exemple les composés du type ester, éther, aldéhyde, cétone, alcool aromatique et hydrocarbure.

Comme esters, on peut citer en particulier l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle.

Comme éthers, on peut citer le benzyléthyléther.

Comme aldéhydes, on peut citer par exemple les alcanals linéaires comportant de 8 à 18 atomes de carbone, le citral, le citronellal, le citronellyloxyacétaldéhyde, le cyclamènaldéhyde, l'hydroxycitronellal, le lilial et le bourgeonal.

Comme cétones, on peut citer par exemple les ionones comme l'alpha-isométhylionone, et la méthylcédrylcétone.

Parmi les alcool aromatiques et notamment terpéniques, on peut citer l'anéthol, le citronellol, l'eugénol, l'isoeugénol, le géraniol, le linalol, le phényléthylalcool et le terpinéol.

Comme hydrocarbures, on peut citer notamment les terpènes. Ces composés se présentent souvent sous forme de mélange de deux ou plus de ces substances odorantes.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'olibian, de galbanum, de labolanum et de lavandin.

On peut utiliser comme substance parfumante, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le linalol, l'ambroxane, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

On peut aussi utiliser un mélange de différentes substances parfumantes qui engendrent en commun une note plaisante pour l'utilisateur. Parmi les notes olfactives connues, on peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères et leurs mélanges.

La quantité de substance(s) parfumante(s) va de 0,5 à 30 % en poids, plus préférentiellement de 0,5 à 10%, encore mieux 0,5 à 5 en poids par rapport au poids total de la composition.

## ACTIFS DEODORANTS

Selon une forme particulière de l'invention, les compositions selon l'invention comprennent en plus au moins un actif déodorant.

Au sens de la présente invention, on entend par "actif déodorant" toute substance capable de masquer, absorber, améliorer ou réduire l'odeur désagréable résultant de la décomposition de la sueur humaine par des bactéries.

Les actifs déodorants peuvent être des agents bactériostatiques ou des agents bactéricides comme le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltrimethylammonium, les sels de cétylpyridinium , le DPTA (acide 1,3-diaminopropanetétraacétique), le 1,2 decanediol (SIMCLARIOL de la société Symrise),

Parmi les actifs déodorants conformes à l'invention, on peut aussi citer également

- les sels de zinc comme le salicylate de zinc, le gluconate de zinc, le pidolate de zinc ; le sulfate de zinc, le chlorure de zinc, le lactate de zinc, le phénolsulfonate de zinc ;
- la chlorhexidine et ses sels;
- le bicarbonate de sodium ;
- l'acide salicylique et ses dérivés tels que l'acide n-octanoyl-5-salicylique
- les dérivés de glycérine comme par exemple le Caprylic/Capric Glycerides (CAPMUL MCM de Abitec), le Caprylate ou caprate de Glycerol (DERMOSOFT GMCY et DERMOSOFT GMC respectivement de STRAETMANS), le Polyglyceryl-2 Caprate (DERMOSOFT DGMC de STRAETMANS)
- les dérivés de biguanide comme les sels de polyhexaméthylène biguanide.

En cas d'incompatibilité ou pour les stabiliser, certains des actifs mentionnés ci-dessus peuvent être incorporés dans des sphérules, notamment des vésicules ioniques ou non-ioniques et/ou des nanoparticules (nanocapsules et/ou nanosphères).

Les actifs déodorants peuvent être présents de préférence dans les compositions selon l'invention dans des concentrations pondérales allant 0,01 à 5% en poids par rapport au poids total de la composition.

## PHASE AQUEUSE

On entend au sens de l'invention, par phase aqueuse par l'eau et l'ensemble des ingrédients de la composition de l'invention solubles dans l'eau.

L'eau variera de préférence dans des concentrations allant de 10 à 40% en poids par rapport au poids total de la composition.

Selon une forme préférentielle de l'invention, la phase aqueuse pourra contenir au moins un monoalcools en $C_1$-$C_4$ et/ou au moins un polyol.

Parmi les monalcools en $C_1$-$C_4$, on citera par exemple le méthanol, l'éthanol, le propanol, l'isopropanol. On utilisera de préférence l'éthanol. Les monoalcools seront présents de préférence à des concentrations allant de 3 à 50% en poids

Parmi les polyols, on peut citer par exemple l'éthylène glycol, la glycérine, l'érythritol, le propylène glycol, le propane1,3-diol, le mannitol, le sorbitol, le xylitol, le maltitol, le lactitol.

Les polyols seront présents de préférence à des concentrations de 40 à 70% en poids par rapport au poids total de la composition.

## ADDITIFS

La composition selon l'invention peut encore contenir d'autres ingrédients bien connus dans le domaine des produits cosmétiques déodorants, dont on peut citer par exemple, des agents apaisants, des conservateurs, des agents antioxydants, des sequestrants, des agents gélifiants ou épaississants hydrophiles, des actifs hydrophiles et leurs mélanges.

Comme agents actifs habituels dans le domaine cosmétique ou dermatologique pouvant être utilisés selon l'invention, on peut citer en particulier tous les actifs connus pour leur activité sur le vieillissement de la peau comme les agents kératolytiques ou prodesquamants, par exemple les α-hydroxy-acides tels que l'acide lactique, l'acide citrique et l'acide glycolique, les β-hydroxy-acides tels que l'acide salicylique et ses dérivés, les α-cétoacides, les β-ceto-acides ; les rétinoïdes et leurs esters, tels que le rétinol et ses esters, le rétinal, les caroténoïdes. On peut citer aussi les 15 vitamines, telles que par exemple les vitamines A, $B_3$, PP, B5, E, K1 et/ou C et les dérivés de ces vitamines et notamment leurs esters ; les agents anti-radicaux libres ; les agents hydratants comme les extraits naturels ; les oligomères procyanidoliques, les hydrolysats de protéines , les dérivés de sucre ; les filtres solaires hydrophiles.

Comme gélifiants, on peut utiliser notamment les gélifiants hydrophiles tels que les polymères carboxyvinyliques, comme les carbomers ; les polyacrylamides et les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Clariant sous la dénomination commerciale « Hostacerin AMPS » (nom CTFA : ammonium polyacryldimethyltauramide) ; ou le copolymère acrylamide /acrylamido-2-methylpropane sulfonate de sodium en émulsion inverse à 40 % dans le polysorbate, commercialisé sous la dénomination SIMULGEL 600 par la société SEPPIC ; les polysaccharides tels que la gomme de xanthane ; et leurs mélanges.

L'invention est illustrée plus en détail dans les exemples suivants. Les quantités sont données en pourcentage massique par rapport au poids total de la composition.

## EXEMPLES

Les compositions solides transparentes déodorantes suivantes ont été réalisées

| INGREDIENTS | Ex1 hors invention sans alcool gras oxyethyléné | Ex2 : invention avec Beheneth-10 | Ex3 Invention avec Beheneth-20 | Ex4 hors invention avec Beheneth-30 | Ex5 hors invention avec Steareth-10 | Ex6 hors invention sans parfum |
|---|---|---|---|---|---|---|
| Actif déodorant | 1 | 1 | 1 | 1 | 1 | 1 |
| EDTA | 0,5 | 0,5 | 0,5 | 0,5 | 0,5 | 0,5 |
| GLYCERIN | 20 | 20 | 20 | 20 | 20 | 20 |
| PROPYLENE GLYCOL | 50 | 50 | 50 | 50 | 50 | 50 |
| SODIUM STEARATE | 5 | 5 | 5 | 5 | 5 | 5 |
| STEARETH-100 | 1 | 1 | 1 | 1 | 1 | 1 |
| BEHENIC ACID | 2 | 2 | 2 | 2 | 2 | 2 |
| PARFUM (FREZENTE SBM 34829 GESXE - FIRMENICH) | 1 | 1 | 1 | 1 | 1 | |
| BEHENETH-10 (EUMELGIN BA-10) | | 1 | | | | |
| BEHENETH-20 (NIKKOL BB-20) | | | 1 | | | |
| BEHENETH-30 (EMALEX BHA-30) | | | | 1 | | |
| STEARETH-10 (BRIJ 76 - CRODA) | | | | | 1 | |
| SODIUM HYDROXIDE | qsp pH | qsp pH | qsp pH | qsp pH | qsp pH | qsp pH |
| EAU | qsp 100 | qsp 100 | qsp 100 | qsp 100 | qsp 100 | qsp 100 |

Les sticks déodorants transparents sont réalisés selon le mode opératoire suivant :

## MODE OPERATOIRE

- On introduit dans un bécher sous agitation la glycérine et le propylène glycol
- On chauffe à 85°C
- On ajoute sous agitation le stéarate de sodium, l'acide béhénique, l'actif déodorant, EDTA, le steareth 100 et l'alcool gras alkoxylé.
- On abaisse la température à 65°C et on ajoute le parfum
- On ajuste l'indice d'acide entre 0 et 0,2mgKOH/g à l'aide de pastilles de soude et on complète en eau.
- On coule à 65°C dans l'article de conditionnement

**TESTS DE STABILITE DE LA TRANSPARENCE**

On réalise sur les formulations 1 à 6, un test de mesure de l'évolution de l'indice d'acide dans le temps et en températures.

Les essais réalisés sont essentiellement des mesures de l'évolution de l'indice d'acide dans le temps et en températures afin de catalyser l'hydrolyse des esters.

**A/ Méthode de mesure de l'indice d'acide** :

La formule de départ est placée sur une plaque chauffante et portée à 66°C l'indice d'acide de départ est alors ajusté entre 0 et 0,1 mg de KOH/g.

Un prélèvement de la solution est fait au bout de 6 heures à 66°C afin d'évaluer l'augmentation d'indice d'acide durant ces 6 heures.

Le dosage de l'indice d'acide est mesuré selon la méthode ci-dessous :

1 - PRINCIPE

L'indice d'acide mesure la quantité de fonction acides libres titrables par une solution d'hydroxyde de sodium.

2 - REACTIFS

- Titrant : NaOH à 0.1N
- Solvant : l'éthanol à 96° est utilisé comme solvant de l'échantillon à doser

3 - MATERIEL

- mémotitrateur type DL 53 METTLER ou équivalent
- électrode : Mettler-Toledo DG111-SC
- récipient de titrage
- burette de volume □10 ml adaptée à la valeur présumée de l'indice

4 - MODE OPERATOIRE

On pèse environ exactement 20 g de la composition solide à doser. On ajoute 60 ml d'éthanol et on solubilise l'échantillon sous agitation et à chaud. On dose le mélange à l'aide de la Solution de NaOH à 0.1N en suivant l'évolution de potentiel On calcule l'indice d'acide selon l'équation suivante :

$$\text{Indice d'acide (mgKOH/g)} = \frac{56{,}1 \times V \times T}{P}$$

avec

P (g) : prise d'essai
V (ml) : volume versé pour l'essai

T : titre réel du titrant utilisé

On détermine l'augmentation de l'indice d'acide par le calcul suivant

Augmentation de l'indice d'acide = Indice d'acide après 6 h - Indice d'acide à t=0

**B/ Méthode de mesure de la transparence** :

Au bout de 6 mois de stockage à 25°C, on mesure la transparence des formulations 1 à 6 avec un appareil 2100P Turbidimeter de la société HACH . Elle est exprimée en Unités de Turbidité Nephélométique (UTN).

RESULTATS

Les résultats sont indiqués dans le tableau suivant :

| | Ex1 hors invention | Ex2 : invention | Ex3 invention | Ex4 hors invention | Ex5 hors invention | Ex6 hors invention sans parfum |
|---|---|---|---|---|---|---|
| **Augmentation de l'indice d'acide après 6 heures (mgKOH/g)** | 0,136 | **0,071** | **0,121** | 0,149 | 0,137 | 0 |
| **Transparence après 6 mois (UTN)** | > 1000 | **< 100** | **< 400** | > 1000 | > 1000 | < 100 |

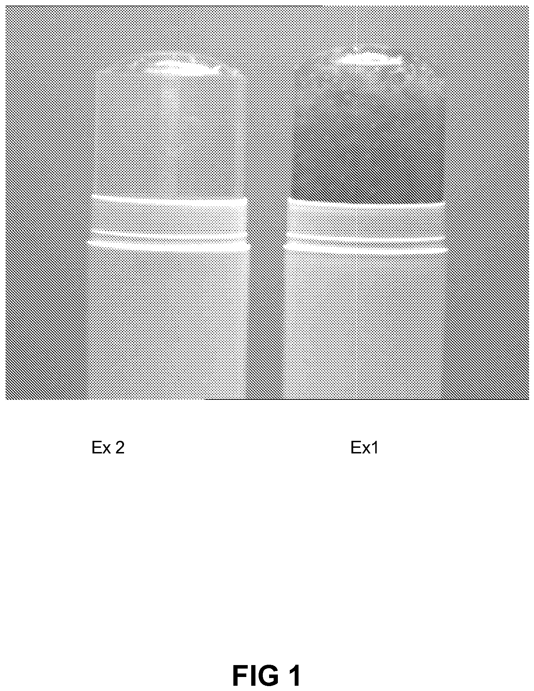

L'aspect des sticks 1 et 2 après 6 mois a été photographié et montré sur la Fig 1.

**Revendications**

1. Composition solide transparente aqueuse parfumante comprenant dans un support cosmétiquement acceptable :

   a) au moins une phase aqueuse et
   b) au moins un sel d'acide gras dans une quantité de 0,5 à 20% en poids par rapport au poids total de la composition et
   c) au moins un alcool béhénique oxyéthyléné de formule générale (I') suivante :

   $$CH_3(CH_2)_{20}\text{-}CH_2\text{-}(OCH_2CH_2)_y\text{-}OH \qquad (I')$$

   dans laquelle y varie de 1 à 20 et plus préférentiellement de 1 à 10, dans une quantité allant de 0,1 à 10% en poids par rapport au poids total de la composition et
   d) au moins une substance parfumante dans une quantité allant de 0,5 à 30% en poids par rapport au poids total de la composition.

2. Composition selon la revendication 1, où le sel d'acide gras est choisi parmi les sels de potassium et les sels de sodium des acides gras en $C_{10}$-$C_{24}$, notamment en $C_{12}$-$C_{20}$, plus spécialement en $C_{12}$-$C_{18}$.

3. Composition selon la revendication 2, où le sel d'acide gras est choisi parmi les sels de sodium des acides gras en $C_{12}$-$C_{18}$, plus spécialement le sel de sodium de l'acide stéarique.

4. Composition selon l'une quelconque des revendications 1 à 3, où la quantité totale de sels d'acide gras varie de 1 à 10 % en poids par rapport au poids total de la composition.

5. Composition selon l'une quelconque des revendication 1 à 4, où l'alcool béhénique oxyéthyléné est le Beheneth-10.

6. Composition selon l'une quelconque des revendications 1 à 5, où la quantité d'alcool gras oxyéthyléné de formule (I') varie de 0,5 à 2% en poids par rapport au poids total de la composition.

7.  Composition selon l'une quelconque des revendications 1 à 6, où la quantité de substance(s) parfumante(s) varie de 0,5 à 10%, encore mieux 0,5 à 5%en poids par rapport au poids total de la composition.

8.  Composition selon l'une quelconque des revendications 1 à 7, comprenant en plus un actif déodorant.

9.  Composition selon l'une quelconque des revendications 1 à 8, dans laquelle l'eau varie dans des concentrations allant de 10 à 40% en poids par rapport au poids total de la composition.

10. Composition selon l'une quelconque des revendications 1 à 9, dans laquelle la phase aqueuse comprend en plus au moins un monoalcool en $C_1$-$C_4$ et/ou au moins un polyol.

11. Composition selon la revendication 10, dans laquelle le ou les monoalcools sont présents à des concentrations allant de 3 à 50% en poids par rapport au poids total de la composition.

12. Composition selon la revendication 11, dans laquelle le ou les polyols sont présents à des concentrations allant de 40 à 70% en poids par rapport au poids total de la composition.

13. Procédé de stabilisation d'une composition solide transparente aqueuse parfumante comprenant dans un support cosmétiquement acceptable :

    a) au moins une phase aqueuse et
    b) au moins un sel d'acide gras tel que défini dans les revendications précédentes dans une quantité de 0,5 à 20% en poids par rapport au poids total de la composition et
    c) au moins une substance parfumante dans une quantité allant de 0,5 à 30% en poids par rapport au poids total de la composition, **caractérisé par le fait qu'**il consiste à ajouter dans ladite composition d) au moins un alcool gras polyoxyéthyléné de formule (I') tel que défini dans les revendications précédentes dans une quantité allant de 0,1 à 10% en poids par rapport au poids total de la composition.

**Patentansprüche**

1.  Feste transparente wässrige Parfümierungszusammensetzung, umfassend in einem kosmetisch unbedenklichen Träger:

    a) mindestens eine wässrige Phase und
    b) mindestens ein Fettsäuresalz in einer Menge von 0,5 bis 20 Gew.-%, bezogen auf das Gesamtgewicht der Zusammensetzung, und
    c) mindestens einen oxyethylenierten Behenylalkohol der folgenden allgemeinen Formel (I'):

    $$CH_3(CH_2)_{20}\text{-}CH_2\text{-}(OCH_2CH_2)_y\text{-}OH \qquad (I')$$

    wobei y von 1 bis 20 und weiter bevorzugt von 1 bis 10 variiert, in einer Menge im Bereich von 0,1 bis 10 Gew.-%, bezogen auf das Gesamtgewicht der Zusammensetzung, und
    d) mindestens eine Parfümierungssubstanz in einer Menge im Bereich von 0,5 bis 30 Gew.-%, bezogen auf das Gesamtgewicht der Zusammensetzung.

2.  Zusammensetzung nach Anspruch 1, wobei das Fettsäuresalz aus den Kaliumsalzen und den Natriumsalzen von $C_{10}$-$C_{24}$-Fettsäuren, insbesondere $C_{12}$-$C_{20}$-Fettsäuren und spezieller $C_{12}$-$C_{18}$-Fettsäuren ausgewählt ist.

3.  Zusammensetzung nach Anspruch 2, wobei das Fettsäuresalz aus den Natriumsalzen von $C_{12}$-$C_{18}$-Fettsäuren ausgewählt ist und spezieller das Natriumsalz von Stearinsäure ist.

4.  Zusammensetzung nach einem der Ansprüche 1 bis 3, wobei die Gesamtmenge an Fettsäuresalzen von 1 bis 10 Gew.-%, bezogen auf das Gesamtgewicht der Zusammensetzung, variiert.

5.  Zusammensetzung nach einem der Ansprüche 1 bis 4, wobei es sich bei dem oxyethylenierten Fettalkohol und Beheneth-10 handelt.

**6.** Zusammensetzung nach einem der Ansprüche 1 bis 5, wobei die Menge an oxyethyleniertem Fettalkohol der Formel (I) von 0,5 bis 2 Gew.-%, bezogen auf das Gesamtgewicht der Zusammensetzung, variiert.

**7.** Zusammensetzung nach einem der Ansprüche 1 bis 6, wobei die Menge an Parfümierungssubstanz bzw. Parfümierungssubstanzen von 0,5 bis 10 Gew.-% und noch besser 0,5 bis 5 Gew.-%, bezogen auf das Gesamtgewicht der Zusammensetzung, variiert.

**8.** Zusammensetzung nach einem der Ansprüche 1 bis 7, die außerdem einen desodorierenden Wirkstoff umfasst.

**9.** Zusammensetzung nach einem der Ansprüche 1 bis 8, wobei das Wasser in Konzentrationen im Bereich von 10 bis 40 Gew.-%, bezogen auf das Gesamtgewicht der Zusammensetzung, variiert.

**10.** Zusammensetzung nach einem der Ansprüche 1 bis 9, wobei die wässrige Phase außerdem mindestens einen $C_1$-$C_4$-Monoalkohol und/oder mindestens ein Polyol umfasst.

**11.** Zusammensetzung nach Anspruch 10, wobei der Monoalkohol bzw. die Monoalkohole in Konzentrationen im Bereich von 3 bis 50 Gew.-%, bezogen auf das Gesamtgewicht der Zusammensetzung, vorliegen.

**12.** Zusammensetzung nach Anspruch 11, wobei das Polyol bzw. die Polyole in Konzentrationen im Bereich von 40 bis 70 Gew.-%, bezogen auf das Gesamtgewicht der Zusammensetzung, vorliegen.

**13.** Verfahren zur Stabilisierung einer festen transparenten wässrigen Parfümierungszusammensetzung, umfassend in einem kosmetisch unbedenklichen Träger:

a) mindestens eine wässrige Phase und
b) mindestens ein Fettsäuresalz gemäß den vorhergehenden Ansprüchen in einer Menge von 0,5 bis 20 Gew.-%, bezogen auf das Gesamtgewicht der Zusammensetzung, und
c) mindestens eine Parfümierungssubstanz in einer Menge im Bereich von 0,5 bis 30 Gew.-%, bezogen auf das Gesamtgewicht der Zusammensetzung,

**dadurch gekennzeichnet, dass** man d) mindestens einen oxyethylenierten Fettalkohol der Formel (I') gemäß den vorhergehenden Ansprüchen in einer Menge im Bereich von 0,1 bis 10 Gew.-%, bezogen auf das Gesamtgewicht der Zusammensetzung, zu der Zusammensetzung gibt.

**Claims**

**1.** Fragrancing aqueous transparent solid composition comprising, in a cosmetically acceptable support:

a) at least one aqueous phase and
b) at least one fatty acid salt in an amount of from 0.5 to 20% by weight relative to the total weight of the composition and
c) at least one oxyethylenated behenyl alcohol of general formula (I') below:

$$CH_3(CH_2)_{20}\text{-}CH_2\text{-}(OCH_2CH_2)_y\text{-}OH \qquad (I')$$

in which y varies from 1 to 20 and more preferably from 1 to 10 in an amount ranging from 0.1 to 10% by weight relative to the total weight of the composition and
d) at least one fragrancing substance in an amount ranging from 0.5 to 30% by weight relative to the total weight of the composition.

**2.** Composition according to Claim 1, where the fatty acid salt is chosen from the potassium salts and the sodium salts of $C_{10}$-$C_{24}$, especially $C_{12}$-$C_{20}$, more especially $C_{12}$-$C_{18}$ fatty acids.

**3.** Composition according to Claim 2, where the fatty acid salt is chosen from the sodium salts of $C_{12}$-$C_{18}$ fatty acids, more especially the sodium salt of stearic acid.

**4.** Composition according to any one of Claims 1 to 3, where the total amount of fatty acid salts varies from 1 to 10% by weight relative to the total weight of the composition.

5. Composition according to any one of Claims 1 to 4, where the oxyethylenated fatty alcohol is Beheneth-10.

6. Composition according to any one of Claims 1 to 5, where the amount of oxyethylenated fatty alcohol of formula (I) varies from 0.5 to 2% by weight relative to the total weight of the composition.

7. Composition according to any one of Claims 1 to 6, where the amount of fragrancing substance(s) varies from 0.5 to 10%, even better still from 0.5 to 5% by weight relative to the total weight of the composition.

8. Composition according to any one of Claims 1 to 7, comprising, in addition, a deodorant active agent.

9. Composition according to any one of Claims 1 to 8, in which the water varies in concentrations ranging from 10 to 40% by weight relative to the total weight of the composition.

10. Composition according to any one of Claims 1 to 9, in which the aqueous phase comprises, in addition, at least one $C_1$-$C_4$ monoalcohol and/or at least one polyol.

11. Composition according to Claim 10, in which the monoalcohol or monoalcohols are present in concentrations ranging from 3 to 50% by weight relative to the total weight of the composition.

12. Composition according to Claim 11, in which the polyol or polyols are present in concentrations ranging from 40 to 70% by weight relative to the total weight of the composition.

13. Method of stabilizing a fragrancing aqueous transparent solid composition comprising, in a cosmetically acceptable support:

a) at least one aqueous phase and
b) at least one fatty acid salt as defined in the preceding claims in an amount of from 0.5 to 20% by weight relative to the total weight of the composition and
c) at least one fragrancing substance in an amount ranging from 0.5 to 30% by weight relative to the total weight of the composition, **characterized by** the fact that it comprises adding, to said composition d) at least one polyoxyethylenated fatty alcohol of formula (I') as defined in the preceding claims in an amount ranging from 0.1 to 10% by weight relative to the total weight of the composition.

Ex 2                          Ex1

# FIG 1

## RÉFÉRENCES CITÉES DANS LA DESCRIPTION

**Documents brevets cités dans la description**

- US 4559924 A
- US 5114717 A
- US 5120541 A
- US 4440742 A
- US 5128123 A
- US 4504465 A
- US 4226889 A
- US 4702916 A
- US 4732754 A
- US 5863524 A
- US 5407668 A